# EUROPEAN PATENT APPLICATION

(11) **EP 4 837 595 A1**
(43) Date of publication of application: **11.11.2026**
(21) Application number: 25175525.2
(22) Date of filing: 09.05.2025
(51) Int. Cl.: A61L 2/07

(54) **PROCESS FOR THE STERILIZATION OF PLANT MATERIAL**

(71) Applicant: W. Kündig & Cie AG, 8006 Zürich (CH)
(72) Inventor: ECKSTEIN, Paul, 98617 Ritschenhausen (DE); GLEICHMANN, Anna Milena, 98617 Ritschenhausen (DE); KRIEG, Marco, 98617 Ritschenhausen (DE); KÜNDIG, Marc-Remo, 8006 Zürich (CH); ROST, Maik, 98617 Ritschenhausen (DE)
(74) Representative: E. Blum & Co. AG

(57) **Abstract**

The present invention relates to a process for the sterilization of plant material, such as cannabis flowers, by exposure to saturated steam. The process provides efficient sterilization while preserving the active and aromatic components of the plant material.

## Description

### Technical field

The present invention relates to a process for the sterilization of plant material, such as cannabis flowers, by exposure to saturated steam. The process provides efficient sterilization while preserving the active and aromatic components of the plant material.

### Background of the invention

The uses of cannabis, mainly its medical uses, tend to grow rapidly. This increases the demand for high-quality cannabis, being safe with a low microbial load, while retaining its sensitive aromatic compounds (mainly terpenes) and physiologically active compounds (cannabinoids).

Irradiation and plasma sterilization methods are extensively used to sterilize cannabis. However, such methods are complex and expensive. In addition, these methods, in particular irradiation, are detrimental to the quality of cannabis, namely in that they impact its colour and cause a reduction in its cannabinoids and terpenes content, thus impacting the flavour and the physiologic effects of the plant. It is therefore sought to provide a method that solves these problems and achieves the same sterilization as irradiation and plasma, while better preserving the colour and sensitive compounds in the treated cannabis. It would be particularly desirable to provide such a method for the sterilization of cannabis flowers.

It would further be advantageous to provides a sterilization method that provides a fresher aspect to the sterilized cannabis, compared to cannabis treated by irradiation or with plasma. It would in particular be advantageous to be able to adjust the residual humidity in the cannabis, such as to obtain sterilized cannabis that retains a similar freshness as the same before treatment.

WO2020/093168A1 discloses methods and apparatus for sterilizing plant materials using cold plasma. The plant material can be cannabis. This document teaches that harsh conditions of steam or gamma radiation results in heating of the cannabis which, in turn alters or destroys active constituents (e.g. cannabinoids or terpenes) and changes the smell, taste, and/or pharmaceutical properties of cannabis sterilized in that manner.

WO2024/028708A1 describes the sterilization of plant material by increasing the humidity to 70-100% in a closed receptacle containing the plant material and heating the receptable for a duration of 0.5 to 60 minutes, while maintaining the relative humidity within the range for at least a portion of the duration. This document also teaches that steam sterilization is avoided, because it causes detachment of the trichomes. The disadvantage of the process described in this document is that it is sub-optimal against spore-forming bacteria and fungi. Also, the process described in this document has the drawback of increasing the humidity of the treated plant material, which can promote mould growth. It is thus desirable to provide a process that achieves a higher level of decontamination by reducing the microbial counts faster and/or more effectively, in particular in terms of reduction of spore-forming bacteriae and fungi. It is further desirable to provide a process that avoids unwanted moisture accumulation in the plant material as a result of the treatment.

WO2021/044167A1 describes an apparatus for steam treating cannabis. This document teaches that the plant material shall not be overheated but requires that the temperature of the steam treatment be of at least 90°C. The treatment conditions are of minimum 90°C for minimum 10 minutes and of maximum 104°C for maximum 30 minutes. This method is disadvantageous in that it causes excessive decarboxylation of tetrahydrocannabinoic acid (THCA) to tetrahydrocannabinol (THC). It is therefore not suitable for THC quality cannabis. It would be desirable to provide a method that successfully sterilized cannabis, while preserving THCA, and that causes reduced decarboxylation. At temperatures of 90°C and higher, the amounts of terpenes also drop, thus modifying the organoleptic properties of the cannabis.

Jerushalemi et al., Effects of steam sterilization on reduction of fungal colony forming units, cannabinoids and terpene levels in medical cannabis inflorescences, Nature (2021)11:13973 discloses exposure of cannabis inflorescences with short pulses of steam for 10, 15 and 20 seconds at 65-70°C (such as 15s at 65°C and 20s at 70°C). This process however provides insufficient microbial decontamination. It is thus desirable to provide a process achieving a better decontamination. It would further be desirable to provide a process that makes it possible to control the humidity of the sterilized product, in particular such as to obtain a similar humidity before and after sterilization.

It is also desirable to provide a process which ensures uniform treatment of the whole batch of contaminated plant material.

It is also desirable to provide a process that makes it possible to control the moisture in the final product.

The present invention aims at solving the above-described problems.

### Summary of the invention

In a first aspect, the invention provides a process for producing a sterilized plant material comprising exposing a plant material to saturated steam at a sterilization temperature of less than 90°C for a holding time of 1 to 30 minutes in a chamber.

In a second aspect, the invention provides a sterilized plant material obtained or obtainable by the process of the present invention.

In a third aspect, the invention provides a process for sterilizing plant material, comprising exposing the plant material to saturated steam at a sterilization temperature of less than 90°C for a holding time of 1 to 30 minutes in a chamber.

### Detailed description of the invention

The present inventors have developed a process for producing sterilized plant materials (in other words for sterilizing plant material), which is suitable for the sterilization of plant materials comprising sensitive flavouring, odorant and/or physiologically active compounds. Such compounds are in particular heat sensitive compounds. The process advantageously achieves effective sterilization of the plant material and retains the sensitive compounds, such as terpenes and cannabinoids, such as tetrahydrocannabinol (THC) and tetrahydrocannabinoic acid (THCA). In particular, the process advantageously provides for very limited decarboxylation of cannabinoid compounds. These advantages are achieved by exposing the plant material to saturated steam within a specific temperature range and for a specific holding time. Thus, the invention provides a process for producing a sterilized plant material comprising exposing the plant material to saturated steam at a sterilization temperature of less than 90°C for a holding time of 1 to 30 minutes in a chamber.

The process can be applied to any plant material. It is however particularly beneficial for sterilizing plant materials comprising sensitive compounds. Thus, in a particular aspect of the invention, the plant material is a plant material comprising terpenes, tetrahydrocannabinol (THC) and/or tetrahydrocannabinoic acid (THCA). Preferably the plant material is of the Cannabaceae family and is more preferably selected from the group consisting of cannabis and hop. In a preferred aspect, the plant material comprises flowers, even more preferably cannabis or hop flowers, most preferably cannabis flowers.

Saturated steam is a particularly effective means for decontaminating plant material. Indeed, the moist heat provided by saturated steam sterilization is particularly effective for denaturation of spore-forming bacteria and fungi and more effective than dry heat at comparable temperatures. Thus, in a preferred aspect, the plant material is a microbially contaminated plant material, preferably a plant material contaminated with spore-forming bacteriae and/or fungi.

For the purpose of the present invention, the term "sterilizing" a plant material refers to reducing the microbial counts in the plant material, preferably reducing the microbial counts to levels that are safe for the intended use of the plant material. In a particular aspect, the term "sterilizing" refers to the reduction of the Total Aerobic Microbial Count (TAMC), the Total Yeast and Mold Count (TYMC), the *Escherichia coli* count, the Salmonella count and/or the Bile-tolerant Gram-negative bacteria count. In a preferred aspect sterilizing refers to reducing the microbial counts of spore-forming bacteria and/or fungi, which are advantageously reduced by the process of the present invention. In a more preferred aspect, the term "sterilizing" refers to:
- reducing the Total Aerobic Microbial Count (TAMC) to 10⁵ cfu/g or less, preferably 10⁴ cfu/g or less;
- reducing the Total Yeast and Mold Count (TYMC) to 10⁴ cfu/g or less, preferably to 10² cfu/g or less;
- reducing the count of bile-tolerant Gram-negative bacteria to 10⁴ cfu/g or less, preferably to 10² cfu/g or less;
- removing Escherichia coli; and/or
- removing Salmonella.

For preserving the sensitive compounds, it is essential to perform the process at a temperature below 90°C. Such temperatures are beneficial in that decarboxylation of cannabinoid acids, such as tetrahydrocannabinoic acid (THCA) is reduced or even avoided, compared to temperatures above 90°C. Thus, the cannabinoids composition is better preserved. Preferably, the sterilization temperature is of 85°C or less, preferably of 82°C or less, more preferably of 80°C or less, more preferably of 75°C or less, most preferably of 72°C or less. The present inventors have demonstrated that a temperature of 82°C was effective in sterilizing the plant material in the presence of extremely high loads of microbial contaminants. Such temperature advantageously preserves a large variety of terpenes, so that the plant material retains its characteristic odour. Temperatures below 80°C, preferably of 75°C or less or 72°C or less advantageously lead to even higher amounts and an even larger diversity of terpenes and provide sufficient sterilization in the presence of average to high microbial contamination. Terpenes like α-pinene, camphene, β-pinene, myrcene, limonene and linalool were better preserved when the plant material, preferably the cannabis plant material, was treated at 72°C. Temperatures below 70°C such as 62°C, are advantageous in that even higher amounts and diversity of terpenes are retained, while being sufficient to sterilize plant materials having low microbial contamination levels. The skilled person can choose the most appropriate sterilization temperature as a function of the level of contamination of the material to be treated, and the desired organoleptic properties of the plant materials.

For the sake of ensuring optimal decontamination, the sterilization temperature is preferably of at least 60°C, preferably at least 62°C. For moderate or high contamination levels the sterilization temperature is preferably of at least 70°C, more preferably at least 72°C.

Also, to optimize the decontamination and to ensure that the whole batch of plant material is decontaminated, it is also preferred that the temperature be evenly distributed within the chamber. Preferably, the temperature varies from one point in the chamber to another point in the chamber by a maximum of 10°C, preferably a maximum of 9°C, preferably a maximum of 8°C, preferably 7°C, more preferably a maximum of 6°C, even more preferably a maximum of 5°C, most preferably a maximum of 4°C. Thus, in a preferred aspect, the sterilization temperature as defined above is the minimum temperature in the chamber, and the temperature at other points of the chamber is comprised between the sterilization temperature and a temperature defined as "sterilization temperature + 10°C", preferably as "sterilization temperature + 9°C", preferably as "sterilization temperature + 8°C", preferably as "sterilization temperature + 7°C", preferably as "sterilization temperature + 6°C", preferably as preferably as "sterilization temperature + 5°C", most preferably as "sterilization temperature + 4°C". In this aspect, the sterilization temperature defines the temperature set for achieving adequate sterilization, while the maximum temperature in the chamber is kept low enough to preserve the sensitive compounds in the plant material. Homogeneity of the temperature in the chamber is advantageous to ensure that the whole plant material batch is homogeneously sterilized and that no portion of the treated material is exposed to excessive heat.

In a preferred aspect of the invention, the temperature in the chamber is monitored during the holding time. Even more preferably, the process is stopped and the chamber is ventilated in case the temperature in the chamber exceeds the set limit. In an even more preferred aspect, the temperature sensor triggers the automatic stopping of the process and the chamber ventilation upon measuring a temperature above a certain value, preferably upon measuring a temperature defined as "sterilization temperature + 10°C", preferably as "sterilization temperature + 9°C", preferably as "sterilization temperature + 8°C", preferably as "sterilization temperature + 7°C", preferably as "sterilization temperature + 6°C", preferably as preferably as "sterilization temperature + 5°C", most preferably as "sterilization temperature + 4°C". These aspects prevent the harmful deterioration of the treated product in case of overheating and ensures that the material present in the chamber retains its active compounds and can thus be re-processed, instead of being discarded.

In a preferred aspect, the holding time is of 1 to 25 minutes, preferably 1 to 20 minutes, preferably 1 to 15 minutes, preferably 1 to 10 minutes, preferably 1 to 5 minutes, preferably 1 to 4 minutes, preferably 1 to 3 minutes, such as 2 minutes. The skilled person can determine the adequate holding time depending on the level of contamination of the plant material, such as for example microbial counts in the untreated plant material.

In a preferred aspect, the plant material is exposed to saturated steam under reduced pressure, preferably under vacuum. Reduced pressure, preferably vacuum, advantageously improves decontamination of the plant material. Reduced pressure, preferably vacuum, improves the distribution of the steam within the chamber and contributes to a uniform temperature within the chamber and to a substantially identical decontamination at all positions of the chamber (i.e. homogeneous decontamination).

When the process is performed under vacuum, the process preferably comprises the following steps:
a) introducing the plant material into the chamber;
b) drawing vacuum in the chamber containing the plant material at least once, preferably at least twice, more preferably at least three times, optionally with the introduction of steam into the chamber between each time vacuum is drawn;
c) introducing saturated steam into the chamber at reduced pressure, preferably under vacuum, at a temperature below the sterilization temperature, and
d) increasing the temperature in the chamber to the sterilization temperature;
e) as soon as the sterilization temperature is reached, maintaining said temperature for the holding time

In step b) air is removed from the chamber. This is more effectively achieved when the vacuum is drawn at least twice and when steam is injected between each time vacuum is drawn. Preferably, the vacuum is drawn at least three times and steam is injected between each time vacuum is drawn. Drawing the vacuum to remove air from the sterilization chamber, as described herein, contributes to the uniformity of the distribution of the steam within the chamber, to the uniformity of the temperature and consequently to the uniformity of the sterilization. It also results in a more efficient steam penetration into the plant material and thus to a more effective sterilization. Drawing the vacuum several times further increases these advantages.

In a preferred aspect, the saturated steam is introduced into the chamber in step c) at a temperature at least 5°C, preferably 5 to 20°C below the sterilization temperature, preferably 5 to 15°C below the sterilization temperature. As the steam is saturated steam (i.e. an ideal gas), the temperature is determined by the pressure in the chamber. In a preferred aspect, the temperature corresponds to the temperature obtained when the chamber is under vacuum, as determined by the ideal gas law. Indeed, it is preferred to start injecting the saturated steam when the chamber is under vacuum. The advantages of injecting the saturated steam under vacuum are the same as those of drawing vacuum several time before the final injection of steam, as defined above.

The temperature in the chamber is then increased to the sterilization temperature after reaching the vacuum. This is preferably performed by increasing the pressure in the chamber containing the saturated steam and the plant material until the sterilization temperature is reached. Preferably the temperature in the chamber is verified by means of at least one temperature sensor in the chamber. As soon as the sterilization temperature is reached, the germ reduction phase begins and the temperature is maintained for the holding time.

Sterilization of the plant material using saturated steam results in a plant material having increased humidity, as steam penetrates the plant material. Penetration of the saturated steam into the plant material is essential to the effective sterilization of the plant material, but it can be detrimental to the appearance and quality of the product, and can lead to recontamination, for example by mould, after the process. Thus, steam sterilization is preferably followed by a drying step. The drying step is preferably performed under reduced pressure, preferably it is vacuum drying. The present inventors have shown that vacuum drying was particularly appropriate to remove the excess moisture in the plant material, while retaining a similar level of humidity as in the plant material provided before exposure to steam (i.e by eliminating the moisture provided by steam during treatment without desiccating the plant material compared to the same material before treatment). This step makes it possible to better control the moisture content in the treated plant material. Thus, in a preferred aspect of the invention, the process further comprises a step of vacuum drying after exposing the plant material to steam.

In a preferred aspect, after the sterilization step e), i.e. as soon as the holding time has ended, the pressure inside the chamber is reduced, such as to reduce the temperature in the chamber to the desired drying temperature. Preferably, vacuum is drawn. The drying phase starts as soon as the drying temperature is reached (preferably as soon a vacuum is drawn), and the drying temperature is maintained for the desired drying time. Due to the low pressure or even the vacuum, and the prevailing reduced temperature, the water from the saturated steam that has been absorbed by the plant material is removed. The advantage of such drying step is that the drying loss exhibits minimal variation compared to the untreated material. After the drying time has elapsed, the chamber is ventilated by introducing filtered air into the chamber.

In a particular aspect of the invention, the drying is performed at a drying temperature below the sterilization temperature, preferably at a temperature 2 to 20°C below the sterilization temperature, preferably 5 to 10°C below the sterilization temperature. In another preferred aspect, the drying step is performed for a drying time of 1 to 30 minutes, preferably 1 to 25 minutes, preferably 1 to 20 minutes, preferably 1 to 15 minutes, preferably 1 to 10 minutes, preferably 1 to 5 minutes, more preferably 2 to 4 minutes, such as 3 minutes.

After the drying step, the vacuum is preferably relieved by introducing air into the chamber so that normal pressure is reached in the chamber, before the chamber can be opened to remove the sterilized plant material.

The plant material obtained or obtainable by the process of the present invention is advantageous in that is has a low microbial load, while having a content in sensitive compounds similar to the content of such compounds in the plant material before the process. Thus, a sterilized plant material obtained or obtainable by the process of the invention is also contemplated as an object of the present invention.

In another aspect, the invention provides a process for sterilizing plant material comprising the steps as described above.

### Examples

### Example 1: Sterilization of hop flowers and assessment of the homogeneity of the temperature in the chamber

Hops blossoms are used for validation of the process to sterilize cannabis, because, like cannabis, they belong to the hemp family (Cannabaceae) and show comparable behaviour in vapor penetration and distribution due to their similar physical properties in terms of structure and moisture content.

The sample preparation was carried out by the Kündig company. For the test, sample bags, which are made of a material permeable to gas and water vapour, were filled with 1,000 g of hops each; care was taken to ensure that there was a filling height of approx. 10 cm. The filling height of approx. 10 cm was selected because this filling height corresponds to the framework of large-scale production. After filling, the bags were closed in such a way that the filling level was maintained during handling.

The germ reduction process was performed in duplicates in two distinct chambers originating from Munich Medicine Mechanics. The samples were placed in the chamber and were subjected to a sterilization process. The sterilization process consisted of the following steps:

**Table 1: sequence of steps of the process of Example 1**

| **Phases** | **Designation** | **Description** |
|---|---|---|
| 1 | Drawing vacuum | To remove the air present in order to obtain an even distribution of vapour. |
| 2 | Steam injection | |
| 3 | Drawing vacuum | |
| 4 | Steam injection | Final steam introduction phase (temperature 59 to 65°C) |
| 5 | Temperture increase | Increasing the pressure until a temperature of 72°C is reached in the chamber |
| 6 | Sterilize | Sterilization phase started as soon as the sterilization temperature of 72°C was reached. The temperature was held in the range 72-77°C for a holding time of 2 minutes. |
| 7 | Pressure relief | Pressure relief of the chamber in preparation for the drying phase, which takes place under vacuum. |
| 8 | Dry | Start of the drying phase by drawing vacuum. Vacuum applied for a drying time of 3 minutes (drying temperature 63 to 68°C). |
| 9 | Ventilate | Filtered air was introduced into the chamber to remove the vacuum and prepare the chamber for relaxation. |
| 10 | Relax | Reaching the pressure area where the chamber door is automatically opened and the process is finished |

Temperature sensors were provided at different positions within the chamber. The measured temperatures throughout the sterilization step remained in both chambers with the prescribed temperature range of 72 to 77°C. Ensuring at the same time optimal sterilization of the sample and also preservation of sensitive compounds, within the whole chamber. Indeed, the lowest measured temperature was in both chambers of 72°C, while the highest measured temperature was of 75.9°C in the first chamber, and of 75.5°C in the second chamber.

### Example 2: Analysis of the cannabinoids and terpenes content in cannabis flowers before and after sterilization according to the present invention at 72°C and 82°C, respectively.

Cannabis flowers were subjected to a saturated steam sterilization process. One paper sample bag, which is made of a material permeable to gas and water vapour, was filled with 60 g of flowers for each Sample. Care was taken to ensure that there was a filling height of approx. 10 cm. The filling height of approx. 10 cm was selected because this filling height corresponds to the framework of large-scale production. After filling, the bags were closed in such a way that the filling level was maintained during handling.

The chamber was filled with 1 kg bags of hops to imitate large-scale production. The same paper sample bags were used. The approx. 60g sample filled with cannabis flowers was placed between these bags.

The germ reduction process was performed in a chamber originating from Munich Medicine Mechanics. The samples were placed in the chamber and were subjected to a sterilization process. The sterilization process consisted of the following steps:

**Table 2: sequence of steps of the process of Example 2**

| **Phases** | **Designation** | **Description** |
|---|---|---|
| 1 | Drawing vacuum | To remove the air present in order to obtain an even distribution of vapour. |
| 2 | Steam injection | |
| 3 | Drawing vacuum | |
| 4 | Steam injection | |
| 5 | Drawing vacuum | |
| 4 | Steam injection | Final steam introduction phase |
| 5 | Temperature increase | Increasing the pressure until the sterilization temperature is reached (see Table 3) |
| 6 | Sterilize | Sterilization phase started as soon as the sterilization temperature was reached. The temperature was held at the sterilization temperature for the holding time (see Table 3). |
| 7 | Pressure relief | Pressure relief of the chamber in preparation for the drying phase, which takes place under vacuum. |
| 8 | Dry | Start of the drying phase by drawing vacuum. Vacuum applied for the drying time (see Table 3). |
| 9 | Ventilate | Filtered air was introduced into the chamber to remove the vacuum and prepare the chamber for relaxation. |
| 10 | Relax | Reaching the pressure area where the chamber door is automatically opened and the process is finished |

**Table 3: Conditions of the process of Example 2**

| | **Sample 01 (untreated)** | **Sample 02 (invention)** | **Sample 03 (invention)** | **Sample 04 (invention)** |
|---|---|---|---|---|
| Sterilization temperature [°C] | n/a | 72 | 72 | 82 |
| Holding time [minute] | n/a | 2 | 2 | 20 |
| Drying time [minute] | n/a | 3 | 3 | 3 |

The contamination of the plant material was then assessed in accordance with the European Pharmacopoeia (paragraph 2.6.12 for TAMC and TYMC and paragraph 2.6.31 for Gram-negative bacteria that tolerate bile salts, E. coli and Salmonella). The results are provided in Table 4.

**Table 4: Contamination of the plant materials obtained after the process of example 2**

| | **Sample 01 (untreated)** | **Sample 02 (invention)** | **Sample 03 (invention)** | **Sample 04 (invention)** |
|---|---|---|---|---|
| TAMC test (cfu/g) | 1.3x10⁴ | <10 | <10 | <10 |
| TYMC test (cfu/g) | 2.2x10⁴ | <10 | <10 | <10 |
| Escherichia coli (per g) | Absent | Absent | Absent | Absent |
| Salmonella (per 25 g) | Absent | Absent | Absent | Absent |
| Gram-negative bacteria that tolerate bile salts (cfu/g) | <10 | <10 | <10 | <10 |
| Aflatoxin B1 | BDL¹⁾ | BDL¹⁾ | BDL¹⁾ | BDL¹⁾ |
| Aflatoxin B2 | BDL¹⁾ | BDL¹⁾ | BDL¹⁾ | BDL¹⁾ |
| Aflatoxin G1 | BDL¹⁾ | BDL¹⁾ | BDL¹⁾ | BDL¹⁾ |
| Aflatoxin G2 | BDL¹⁾ | BDL¹⁾ | BDL¹⁾ | BDL¹⁾ |
| Combined aflatoxins | BDL¹⁾ | BDL¹⁾ | BDL¹⁾ | BDL¹⁾ |

| | | | | |
|---|---|---|---|---|
| 1) Below the Detection Limit | | | | |

The microbial decontamination was very effective for all samples of the invention.

The amounts of cannabinoid compounds were determined in accordance with the European Pharmacopoeia (paragraph 2.2.29). The results are provided in Table 5.

**Table 5: Cannabinoids content in the plant materials sterilized in Example 2**

| | **Sample 01 (untreated)** | **Sample 02 (invention)** | **Sample 03 (invention)** | **Sample 04 (invention)** |
|---|---|---|---|---|
| Cannabinol (CBN) [% m/m] | 0.01 | 0.01 | 0.01 | 0.04 |
| (-)-trans-Δ⁹-tetrahydrocannabinol (Δ⁹-THC) [% m/m] | 0.67 | 1.06 | 1.14 | 8.14 |
| Tetrahydrocannabinolic acid (THCA) [% m/m] | 26.80 | 26.86 | 26.51 | 18.72 |
| Cannabidiol (CBD) [% m/m] | n/d¹⁾ | n/d¹⁾ | n/d¹⁾ | n/d¹⁾ |
| Cannabidiolic acid [% m/m] | 0.11 | 0.10 | 0.10 | 0.10 |
| Total Δ⁹-THC+ THCA [% m/m] | 27.47 % (m/m) | 27.92 % (m/m) | 27.65 % (m/m) | 26.86 % (m/m) |
| Total CBD + cannabidiolic acid [% m/m] | 0.11 % (m/m) | 0.10 % (m/m) | 0.10 % (m/m) | 0.10% (m/m) |

| | | | | |
|---|---|---|---|---|
| 1) Not detected | | | | |

Very low decarboxylation of THCA was observed in Samples 02 and 03 of the invention (respectively treated at 62 and 72°C, while limited decarboxylation was observed in Sample 04 (treated at 82°C). Cannabidiolic acid was also successfully retained.

The amounts of terpenes were determined in accordance with the European Pharmacopoeia (paragraph 2.2.28). The results are provided in Table 6.

**Table 6: Terpenes content in the plant materials sterilized in Example 2**

| Terpenes | **Sample 01 (untreated)** | **Sample 02 (invention)** | **Sample 03 (invention)** | **Sample 04 (invention)** |
|---|---|---|---|---|
| α-Pinene [% m/m] | 0.05 | 0.02 | 0.01 | BDL¹⁾ |
| Camphene [% m/m] | 0.01 | 0.01 | 0.01 | n/d²⁾ |
| β-Pinene [% m/m] | 0.09 | 0.03 | 0.03 | BDL¹⁾ |
| Myrcene[% m/m] | 0.14 | 0.03 | 0.02 | BDL¹⁾ |
| Δ-3- Carene [% m/m] | n/d²⁾ | n/d²⁾ | n/d²⁾ | n/d²⁾ |
| α-Terpinene [% m/m] | BDL¹⁾ | n/d²⁾ | n/d²⁾ | n/d²⁾ |
| p-Cymene [% m/m] | n/d²⁾ | n/d²⁾ | n/d²⁾ | n/d²⁾ |
| Limonene[% m/m] | 0.45 | 0.12 | 0.09 | 0.01 |
| Eucalyptol (1,8-Cineol) [% m/m] | BDL¹⁾ | BDL¹⁾ | n/d²⁾ | n/d²⁾ |
| α-Ocimene [% m/m] | BDL¹⁾ | n/d²⁾ | n/d²⁾ | n/d²⁾ |
| β-Ocimene [% m/m] | BDL¹⁾ | n/d²⁾ | BDL¹⁾ | n/d²⁾ |
| γ-Terpinene [% m/m] | BDL¹⁾ | n/d²⁾ | n/d²⁾ | n/d²⁾ |
| Terpinolene [% m/m] | 0.01 | 0.01 | BDL¹⁾ | BDL¹⁾ |
| Linalool [% m/m] | 0.26 | 0.19 | 0.18 | 0.13 |
| Isopulegol [% m/m] | n/d²⁾ | n/d²⁾ | n/d²⁾ | BDL¹⁾ |
| Geraniol [% m/m] | 0.02 | 0.01 | 0.03 | 0.01 |
| β-Caryophyllene [% m/m] | 0.27 | 0.20 | 0.22 | 0.17 |
| α-Humulene [% m/m] | 0.08 | 0.06 | 0.06 | 0.05 |
| cis-Nerolidol [% m/m] | 0.03 | 0.02 | 0.02 | 0.02 |
| trans-Nerolidol [% m/m] | 0.05 | 0.05 | 0.05 | 0.04 |
| Caryophyllenoxyde [% m/m] | 0.02 | 0.02 | 0.02 | 0.01 |
| Guaiol [% m/m] | 0.10 | 0.09 | 0.10 | 0.10 |
| α-Bisabolol [% m/m] | 0.03 | 0.03 | 0.03 | 0.03 |
| Total | 1.61 | 0.89 | 0.87 | 0.66 |

| | | | | |
|---|---|---|---|---|
| 1) Below the Detection Limit 2) Not detected | | | | |

These results demonstrate that all terpenes present in the un-treated material were still detected in the cannabis flowers treated at 72°C (Samples 01 and 02), even though the amounts of a few terpenes were reduced in Sample 02 (such as α-pinene, β-pinene, myrcene, limonene and linalool). A few terpenes were present in lower amounts at a sterilization temperature of 82°C (Sample 03), such as α-pinene, camphene, β-pinene, myrcene, terpinolene, limonene and linalool. All samples presented the characteristic odour of cannabis.

The drying loss was determined in accordance with the European Pharmacopoeia (paragraph 2.2.32). the drying loss of Samples 01, 02, 03 and 04 was respectively of 6.64%, 6.04%, 5.78% and 5.45%.

### Example 3: Analysis of the cannabinoids and terpenes content in cannabis flowers before and after sterilization according to the present invention at 60°C.

Cannabis flowers were subjected to a saturated steam sterilization process. One paper sample bag, which is made of a material permeable to gas and water vapour, was filled with 60 g of flowers for each Sample. Care was taken to ensure that there was a filling height of approx. 10 cm. The filling height of approx. 10 cm was selected because this filling height corresponds to the framework of large-scale production. After filling, the bags were closed in such a way that the filling level was maintained during handling.

The chamber was filled with 1 kg bags of hops to imitate large-scale production. The same paper sample bags were used. The approx. 60g sample filled with cannabis flowers was placed between these bags.

The germ reduction process was performed in a chamber originating from Munich Medicine Mechanics. The samples were placed in the chamber and were subjected to a sterilization process. The sterilization process consisted of the following steps:

**Table 7: sequence of steps of the process of Example 3**

| **Phases** | **Designation** | **Description** |
|---|---|---|
| 1 | Drawing vacuum | To remove the air present in order to obtain an even distribution of vapour. |
| 2 | Steam injection | |
| 3 | Drawing vacuum | |
| 4 | Steam injection | |
| 5 | Drawing vacuum | |
| 4 | Steam injection | Final steam introduction phase |
| 5 | Temperature increase | Increasing the pressure until the sterilization temperature is reached (see Table 3) |
| 6 | Sterilize | Sterilization phase started as soon as the sterilization temperature was reached. The temperature was held at the sterilization temperature for the holding time (see Table 3). |
| 7 | Pressure relief | Pressure relief of the chamber in preparation for the drying phase, which takes place under vacuum. |
| 8 | Dry | Start of the drying phase by drawing vacuum. Vacuum applied for the drying time (see Table 3). |
| 9 | Ventilate | Filtered air was introduced into the chamber to remove the vacuum and prepare the chamber for relaxation. |
| 10 | Relax | Reaching the pressure area where the chamber door is automatically opened and the process is finished |

**Table 8: Conditions of the process of Example 3**

| | **Sample 05 (untreated)** | **Sample 06 (invention)** |
|---|---|---|
| Sterilization temperature [°C] | n/a | 60 |
| Holding time [minute] | n/a | 2 |
| Drying time [minute] | n/a | 3 |

The contamination of the plant material was then assessed in accordance with the European Pharmacopoeia (paragraph 2.6.12 for TAMC and TYMC and paragraph 2.6.31 for Gram-negative bacteria that tolerate bile salts, E. coli and Salmonella). The results are provided in Table 9.

**Table 9: Contamination of the plant materials obtained after the process of example 3**

| | **Sample 05 (untreated)** | **Sample 06 (invention)** |
|---|---|---|
| TAMC test (cfu/g) | <1000 | <1000 |
| TYMC test (cfu/g) | 70 | <10 |
| Escherichia coli (per g) | Absent | Absent |
| Salmonella (per 25 g) | Absent | Absent |
| Gram-negative bacteria that tolerate bile salts (cfu/g) | >10 and <100 | <10 |
| Aflatoxin B1 | BDL¹⁾ | BDL¹⁾ |
| Aflatoxin B2 | BDL¹⁾ | BDL¹⁾ |
| Aflatoxin G1 | BDL¹⁾ | BDL¹⁾ |
| Aflatoxin G2 | BDL¹⁾ | BDL¹⁾ |
| Combined aflatoxins | BDL¹⁾ | BDL¹⁾ |

| | | |
|---|---|---|
| 2) Below the Detection Limit | | |

The microbial decontamination was effective in the sample of the invention.

The amounts of cannabinoid compounds were determined in accordance with the European Pharmacopoeia (paragraph 2.2.29). The results are provided in Table 10.

**Table 10: Cannabinoids content in the plant materials sterilized in Example 3**

| | **Sample 05 (untreated)** | **Sample 06 (invention)** |
|---|---|---|
| Cannabinol (CBN) [g/100g] | <0.1 | <0.1 |
| (-)-trans-Δ⁹-tetrahydrocannabinol (Δ⁹-THC) [g/100g] | 0.2 | 0.4 |
| Tetrahydrocannabinolic acid (THCA) [g/100g] | 22.5 | 23.4 |
| Cannabidiol (CBD) [g/100g] | <0.1 | <0.1 |
| Cannabidiolic acid [g/100g] | <0.1 | <0.1 |
| Total Δ⁹-THC+ THCA [g/100g] | 22.7 | 23.8 |
| Total CBD + cannabidiolic acid [g/100g] | <0.1 | <0.1 |

No decarboxylation of THCA and of cannabidiolic acid was observed in the sample of the invention.

The amounts of terpenes were determined in accordance with the European Pharmacopoeia (paragraph 2.2.28). The results are provided in Table 11.

**Table 11: Terpenes content in the plant materials sterilized in Example 3**

| **Terpenes** | **Sample 05 (untreated)** | **Sample 06 (invention)** |
|---|---|---|
| α-Pinene [mg/g] | 0.12 | 0.12 |
| Camphene [mg/g] | 0.034 | 0.032 |
| β-Pinene [mg/g] | 0.24 | 0.23 |
| Myrcene[mg/g] | 1.8 | 1.4 |
| Δ-3- Carene [mg/g] | BDL¹⁾ | BDL¹⁾ |
| α-Terpinene [mg/g] | BDL¹⁾ | BDL¹⁾ |
| p-Cymene [mg/g] | BDL¹⁾ | BDL¹⁾ |
| Limonene [mg/g] | 1.1 | 0.98 |
| Eucalyptol (1,8-Cineol) [mg/g] | 0.075⁾ | 0.077 |
| α-Ocimene [mg/g] | 0.011 | 0.029 |
| β-Ocimene [mg/g] | | |
| γ-Terpinene [mg/g] | 0.011 | BDL¹⁾ |
| Terpinolene [mg/g] | 0.019 | 0.015 |
| Linalool [mg/g] | 1.7 | 1.9 |
| Isopulegol [mg/g] | BDL¹⁾ | BDL¹⁾ |
| Geraniol [mg/g] | BDL¹⁾ | BDL¹⁾ |
| β-Caryophyllene [mg/g] | 2.9 | 3.9 |
| α-Humulene [mg/g] | 1.1 | 1.2 |
| cis-Nerolidol [mg/g] | 1.3 | 1.2 |
| trans-Nerolidol [mg/g] | | |
| Caryophyllenoxyde [mg/g] | 0.076 | 0.11 |
| Guaiol [mg/g] | 1.7 | 1.9 |
| α-Bisabolol [mg/g] | 1.3 | 1.2 |
| Total terpenes [mg/g] | 13 | 14 |
| 1) Below the Detection Limit | | |

These results demonstrate that all terpenes and cannabinoid compounds present in the un-treated material were successfully retained in the cannabis flowers treated at 60°C (Sample 06) at a similar level as in the untreated material. The overall terpenes content was retained in the samples treated according to the invention and all samples presented the characteristic odour of cannabis. At the same time, it is demonstrated that a temperature of 60°C was sufficient to achieve proper decontamination of the cannabis flowers, thus showing that such temperature is appropriate in case of low levels of contaminants in the materials to be treated.

The drying loss was determined in accordance with the European Pharmacopoeia (paragraph 2.2.32). the drying loss of Samples 05 and 06 was respectively of 3.3% and 3.4.

### Example 4: Analysis of the cannabinoids and terpenes content in cannabis flowers before and after sterilization according to the present invention at 60°C.

Cannabis flowers were subjected to a saturated steam sterilization process. One paper sample bag, which is made of a material permeable to gas and water vapour, was filled with 60 g of flowers for each Sample. Care was taken to ensure that there was a filling height of approx. 10 cm. The filling height of approx. 10 cm was selected because this filling height corresponds to the framework of large-scale production. After filling, the bags were closed in such a way that the filling level was maintained during handling.

The chamber was filled with 1 kg bags of hops to imitate large-scale production. The same paper sample bags were used. The approx. 60g sample filled with cannabis flowers was placed between these bags.

The germ reduction process was performed in a chamber originating from Munich Medicine Mechanics. The samples were placed in the chamber and were subjected to a sterilization process. The sterilization process consisted of the following steps:

**Table 12: sequence of steps of the process of Example 4**

| **Phases** | **Designation** | **Description** |
|---|---|---|
| 1 | Drawing vacuum | To remove the air present in order to obtain an even distribution of vapour. |
| 2 | Steam injection | |
| 3 | Drawing vacuum | |
| 4 | Steam injection | |
| 5 | Drawing vacuum | |
| 4 | Steam injection | Final steam introduction phase |
| 5 | Temperature increase | Increasing the pressure until the sterilization temperature is reached (see Table 3) |
| 6 | Sterilize | Sterilization phase started as soon as the sterilization temperature was reached. The temperature was held at the sterilization temperature for the holding time (see Table 3). |
| 7 | Pressure relief | Pressure relief of the chamber in preparation for the drying phase, which takes place under vacuum. |
| 8 | Dry | Start of the drying phase by drawing vacuum. Vacuum applied for the drying time (see Table 3). |
| 9 | Ventilate | Filtered air was introduced into the chamber to remove the vacuum and prepare the chamber for relaxation. |
| 10 | Relax | Reaching the pressure area where the chamber door is automatically opened and the process is finished |

**Table 13: Conditions of the process of Example 4**

| | **Sample 07 (untreated)** | **Sample 08 (invention)** |
|---|---|---|
| Sterilization temperature [°C] | n/a | 60 |
| Holding time [minute] | n/a | 2 |
| Drying time [minute] | n/a | 3 |

The contamination of the plant material was then assessed in accordance with the European Pharmacopoeia (paragraph 2.6.12 for TAMC and TYMC and paragraph 2.6.31 for Gram-negative bacteria that tolerate bile salts, E. coli and Salmonella). The results are provided in Table 14.

**Table 14: Contamination of the plant materials obtained after the process of Example 4**

| | **Sample 07 (untreated)** | **Sample 08 (invention)** |
|---|---|---|
| TAMC test (cfu/g) | <1000 | <1000 |
| TYMC test (cfu/g) | >500 | <10 |
| Escherichia coli (per g) | Absent | Absent |
| Salmonella (per 25 g) | Absent | Absent |
| Gram-negative bacteria that tolerate bile salts (cfu/g) | <10 | <10 |
| Aflatoxin B1 | BDL¹⁾ | BDL¹⁾ |
| Aflatoxin B2 | BDL¹⁾ | BDL¹⁾ |
| Aflatoxin G1 | BDL¹⁾ | BDL¹⁾ |
| Aflatoxin G2 | BDL¹⁾ | BDL¹⁾ |
| Combined aflatoxins | BDL¹⁾ | BDL¹⁾ |

| | | |
|---|---|---|
| 1) Below the Detection Limit | | |

The microbial decontamination was effective for all samples of the invention.

The amounts of cannabinoid compounds were determined in accordance with the European Pharmacopoeia (paragraph 2.2.29). The results are provided in Table 15.

**Table 15: Cannabinoids content in the plant materials sterilized in Example 4**

| | **Sample 07 (untreated)** | **Sample 08 (invention)** |
|---|---|---|
| Cannabinol (CBN) [g/100g] | <0,01 | <0,01 |
| | | |
| (-)-trans-Δ⁹-tetrahydrocannabinol (Δ⁹-THC) [g/100g] | 0,2 | 0.3 |
| Tetrahydrocannabinolic acid (THCA) [g/100g] | 20.7 | 20.0 |
| Cannabidiol (CBD) [g/100g] | <0.1 | <0.1 |
| Cannabidiolic acid [g/100g] | <0.1 | <0.1 |
| Total Δ⁹-THC+ THCA [g/100g] | 20.9 | 20.3 |
| Total CBD + cannabidiolic acid [g/100g] | <0.1 | <0.1 |

Very low decarboxylation of THCA was observed in the sample of the invention, despite the steam treatment.

The amounts of terpenes were determined in accordance with the European Pharmacopoeia (paragraph 2.2.28). The results are provided in Table 16.

**Table 16: Terpenes content in the plant materials sterilized in Example 4**

| **Terpenes** | **Sample 07 (untreated)** | **Sample 08 (invention)** |
|---|---|---|
| α-Pinene [mg/g] | 0.15 | 0.068 |
| Camphene [mg/g] | 0.041 | 0.019 |
| β-Pinene [mg/g] | 0.29 | 0.13 |
| Myrcene[mg/g] | 2.0 | 0.48 |
| Δ-3- Carene [mg/g] | BDL¹⁾ | BDL¹⁾ |
| α-Terpinene [mg/g] | BDL¹⁾ | BDL¹⁾ |
| ρ-Cymene [mg/g] | BDL¹⁾ | BDL¹⁾ |
| Limonene [mg/g] | 0.89 | 0.26 |
| Eucalyptol (1,8-Cineol) [mg/g] | 0.093 | 0.076 |
| α-Ocimene [mg/g] | 0.017 | 0.014 |
| β-Ocimene [mg/g] | | |
| γ-Terpinene [mg/g] | 0.011 | BDL¹⁾ |
| Terpinolene [mg/g] | 0.013 | BDL¹⁾ |
| Linalool [mg/g] | 1.3 | 0.88 |
| Isopulegol [mg/g] | BDL¹⁾ | BDL¹⁾ |
| Geraniol [mg/g] | BDL¹⁾ | BDL¹⁾ |
| β-Caryophyllene [mg/g] | 3.6 | 3.1 |
| α-Humulene [mg/g] | 1.2 | 0.98 |
| cis-Nerolidol [mg/g] | 1.4 | 1.2 |
| trans-Nerolidol [mg/g] | | |
| Caryophyllenoxyde [mg/g] | 0.11 | 0.18 |
| Guaiol [mg/g] | 1.8 | 1.5 |
| α-Bisabolol [mg/g] | 1.3 | 1.2 |
| Total terpenes [mg/g] | 13 | 10 |

| | | |
|---|---|---|
| 1) Below the Detection Limit | | |

These results demonstrate that almost all terpenes present in the un-treated material were still detected in the cannabis flowers treated at 60°C (Sample 08). Only γ-Terpinene and terpinolene were not detectable in Sample 08. Even though the amounts of several terpenes were slightly reduced, the overall terpenes content was retained in the samples treated according to the invention and all samples presented the characteristic odour of cannabis.

The drying loss was determined in accordance with the European Pharmacopoeia (paragraph 2.2.32). the drying loss of the untreated sample (Sample 07) and of Sample 08 of the invention was respectively of 9.3% and 7.3%.

### Example 5: Analysis of the cannabinoids and terpenes content in cannabis flowers before and after sterilization according to the present invention at 62°C, 72°C and 82°C.

Cannabis flowers were subjected to a saturated steam sterilization process. One paper sample bag, which is made of a material permeable to gas and water vapour, was filled with 60 g of flowers for each Sample. Care was taken to ensure that there was a filling height of approx. 10 cm. The filling height of approx. 10 cm was selected because this filling height corresponds to the framework of large-scale production. After filling, the bags were closed in such a way that the filling level was maintained during handling.

The chamber was filled with 1 kg bags of hops to imitate large-scale production. The same paper sample bags were used. The approx. 60g sample filled with cannabis flowers was placed between these bags.

The germ reduction process was performed in a chamber originating from Munich Medicine Mechanics. The samples were placed in the chamber and were subjected to a sterilization process. The sterilization process consisted of the following steps:

**Table 17: sequence of steps of the process of Example 5**

| **Phases** | **Designation** | **Description** |
|---|---|---|
| 1 | Drawing vacuum | To remove the air present in order to obtain an even distribution of vapour. |
| 2 | Steam injection | |
| 3 | Drawing vacuum | |
| 4 | Steam injection | |
| 5 | Drawing vacuum | |
| 4 | Steam injection | Final steam introduction phase |
| 5 | Temperature increase | Increasing the pressure until the sterilization temperature is reached (see Table 3) |
| 6 | Sterilize | Sterilization phase started as soon as the sterilization temperature was reached. The temperature was held at the sterilization temperature for the holding time (see Table 3). |
| 7 | Pressure relief | Pressure relief of the chamber in preparation for the drying phase, which takes place under vacuum. |
| 8 | Dry | Start of the drying phase by drawing vacuum. Vacuum applied for the drying time (see Table 3). |
| 9 | Ventilate | Filtered air was introduced into the chamber to remove the vacuum and prepare the chamber for relaxation. |
| 10 | Relax | Reaching the pressure area where the chamber door is automatically opened and the process is finished |

**Table 18: Conditions of the process of Example 5**

| | **Sample 09 (untreat.)** | **Sample 10 (inv.)** | **Sample 11 (inv.)** | **Sample 12 (inv.)** | **Sample 13 (inv.)** | **Sample 14 (inv.)** |
|---|---|---|---|---|---|---|
| Sterilization temperature [°C] | n/a | 62 | 62 | 62 | 72 | 82 |
| Holding time [minute] | n/a | 2 | 4 | 8 | 2 | 2 |
| Drying time [minute] | n/a | 3 | 3 | 3 | 3 | 3 |

The contamination of the plant material was then assessed in accordance with the European Pharmacopoeia (paragraph 2.6.12 for TAMC and TYMC and paragraph 2.6.31 for Gram-negative bacteria that tolerate bile salts, E. coli and Salmonella). The results are provided in Table 19.

**Table 19: Contamination of the plant materials obtained after the process of example 5**

| | **Sample 09 (untreat.)** | **Sample 10 (inv.)** | **Sample 11 (inv.)** | **Sample 12 (inv.)** | **Sample 13 (inv.)** | **Sample 14 (inv.)** |
|---|---|---|---|---|---|---|
| TAMC test (cfu/g) | 1900 | 1700 | 280 | 1700 | 190 | 180 |
| TYMC test (cfu/g) | 2300 | <10 | 10 | 90 | <10 | <10 |
| Escherichia coli (per g) | Absent | Absent | Absent | Absent | Absent | Absent |
| Salmonella (per 25 g) | Absent | Absent | Absent | Absent | Absent | Absent |
| Gram-negative bacteria that tolerate bile salts (cfu/g) | <1000 and >100 | <100 and >10 | <100 and >10 | <10 | <100 and >10 | <10 |

The microbial decontamination was effective for all samples of the invention.

The amounts of cannabinoid compounds were determined in accordance with the European Pharmacopoeia (paragraph 2.2.29). The results are provided in Table 20.

**Table 20: Cannabinoids content in the plant materials sterilized in Example 5**

| | **Sample 09 (untreat.)** | **Sample 10 (inv.)** | **Sample 11 (inv.)** | **Sample 12 (inv.)** | **Sample 13 (inv.)** | **Sample 14 (inv.)** |
|---|---|---|---|---|---|---|
| Cannabinol (CBN) [% m/m] | 0.04 | 0.04 | 20.04 | 0.04 | 0.04 | 0.05 |
| (-)-trans-Δ⁹-tetrahydrocannabinol (Δ⁹-THC) [% m/m] | 3.03 | 3.01 | 2.96 | 3.16 | 3.06 | 4.16 |
| Tetrahydrocannabinolic acid (THCA) [% m/m] | 18.60 | 19.16 | 19.49 | 18.70 | 18.25 | 18.91 |
| Cannabidiol (CBD) [% m/m] | BDL¹⁾ | BDL¹⁾ | BDL¹⁾ | BDL¹⁾ | BDL¹⁾ | BDL¹⁾ |
| Cannabidiolic acid [% m/m] | 0.05 | 0.05 | 0.05 | 0.05 | 0.05 | 0.06 |
| Total Δ⁹-THC+ THCA [% m/m] | 21.63 | 22.16 | 22.44 | 21.86 | 21.31 | 23.07 |
| Total CBD + cannabidiolic acid [% m/m] | 0.05 | 0.05 | 0.05 | 0.05 | 0.05 | 0.06 |

| | | | | | | |
|---|---|---|---|---|---|---|
| 1) Below detection level | | | | | | |

No decarboxylation of THCA and cannabidiolic acid was observed in the samples of the invention.

The amounts of terpenes were determined in accordance with the European Pharmacopoeia (paragraph 2.2.28). The results are provided in Table 21.

**Table 21: Terpenes content in the plant materials sterilized in Example 5**

| | **Sample 09 (untreat.)** | **Sample 10 (inv.)** | **Sample 11 (inv.)** | **Sample 12 (inv.)** | **Sample 13 (inv.)** | **Sample 14 (inv.)** |
|---|---|---|---|---|---|---|
| α-Pinene [% m/m] | 0.099 | 0.039 | 0.030 | 0.027 | 0.019 | 0.017 |
| Camphene [% m/m] | 0.007 | n/d²⁾ | BDL¹⁾ | BDL¹⁾ | n/d²⁾ | BDL¹⁾ |
| β-Pinene [% m/m] | 0.082 | 0.036 | 0.031 | 0.026 | 0.020 | 0.021 |
| Myrcene[% m/m] | 0.108 | 0.029 | 0.024 | 0.019 | 0.015 | 0.015 |
| Δ-3- Carene [% m/m] | 0.019 | 0.007 | 0.006 | BDL¹⁾ | 0.005 | 0.005 |
| α-Terpinene [% m/m] | 0.010 | 0.007 | 0.007 | 0.007 | 0.006 | 0.006 |
| p-Cymene [% m/m] | BDL¹⁾ | BDL¹⁾ | BDL¹⁾ | BDL¹⁾ | BDL¹⁾ | BDL¹⁾ |
| Limonene [% m/m] | 0.109 | 0.041 | 0.038 | 0.031 | 0.028 | 0.028 |
| Eucalyptol (1,8-Cineol) [% m/m] | 0.007 | 0.005 | 0.007 | 0.005 | 0.005 | 0.005 |
| α-Ocimene [% m/m] | n/d²⁾ | n/d²⁾ | n/d²⁾ | n/d²⁾ | n/d²⁾ | n/d²⁾ |
| β-Ocimene [% m/m] | 0.035 | 0.010 | 0.010 | BDL¹⁾ | BDL¹⁾ | BDL¹⁾ |
| γ-Terpinene [% m/m] | 0.016 | 0.007 | 0.007 | 0.009 | 0.007 | 0.008 |
| Terpinolene [% m/m] | 0.149 | 0.055 | 0.048 | 0.045 | 0.036 | 0.041 |
| Linalool [% m/m] | BDL¹⁾ | BDL¹⁾ | BDL¹⁾ | BDL¹⁾ | BDL¹⁾ | BDL¹⁾ |
| Isopulegol [% m/m] | 0.012 | BDL¹⁾ | BDL¹⁾ | BDL¹⁾ | BDL¹⁾ | 0.010 |
| Geraniol [% m/m] | 0.008 | 0.008 | 0.009 | 0.008 | 0.008 | 0.009 |
| β-Caryophyllene [% m/m] | 0.105 | 0.096 | 0.088 | 0.097 | 0.094 | 0.093 |
| α-Humulene [% m/m] | 0.048 | 0.046 | 0.043 | 0.047 | 0.044 | 0.045 |
| cis-Nerolidol [% m/m] | 0.014 | 0.017 | 0.016 | 0.015 | 0.016 | 0.012 |
| trans-Nerolidol [% m/m] | 0.038 | 0.041 | 0.039 | 0.040 | 0.042 | 0.039 |
| Caryophyllenoxy de [% m/m] | 0.017 | 0.021 | 0.020 | 0.014 | 0.018 | 0.013 |
| Guaiol [% m/m] | BDL¹⁾ | BDL¹⁾ | BDL¹⁾ | BDL¹⁾ | BDL¹⁾ | BDL¹⁾ |
| α-Bisabolol [% m/m] | 0.051 | 0.056 | 0.055 | 0.061 | 0.059 | 0.056 |

| | | | | | | |
|---|---|---|---|---|---|---|
| 1) Below the Detection Limit 2) Not detected | | | | | | |

These results demonstrate that most terpenes present in the un-treated material were still detected in the cannabis flowers treated at 62°C (Samples 10, 11 and 12), 72°C (Sample 13) and 82°C (Sample 14), even though the amounts of several terpenes were reduced. The terpenes amounts were reduced to a larger extent when increasing the sterilization temperature or the holding time. However, increasing the temperature or the holding time resulted in improvements if the sterilization efficiency. Increasing the temperature has proved to better increase the sterilization efficiency than increasing the holding time, because samples 13 and 14 (respectively treated at 72 and 82°C) had less residual contamination compared to samples 11 and 12 treated for a longer time at 62°C). The overall terpenes content was retained in the samples treated according to the invention and all samples presented the characteristic odour of cannabis. This demonstrates that even temperatures of 82°C make it possible to retain the desired organoleptic properties of cannabis.

The drying loss was determined in accordance with the European Pharmacopoeia (paragraph 2.2.32). the drying loss of Samples 09 to 14 was respectively of 6.48%, 5.67%, 5.51%, 5.66%, 5.20% and 5.08%.

### Example 6: Analysis of the cannabinoids and terpenes content in cannabis flowers before and after sterilization according to the present invention at 60°C.

Cannabis flowers were subjected to a saturated steam sterilization process. Sample paper bags, which are permeable to gas and water vapour, were filled with 1,000 g of flowers each; care was taken to ensure that there was a filling height of approx. 10 cm. The filling height of approx. 10 cm was selected because this filling height corresponds to the framework of large-scale production. After filling, the bags were closed in such a way that the filling level was maintained during handling.

The germ reduction process was performed in a chamber originating from Munich Medicine Mechanics. The samples were placed in the chamber and were subjected to a sterilization process. The sterilization process consisted of the following steps:

**Table 22: sequence of steps of the process of Example 6**

| **Phases** | **Designation** | **Description** |
|---|---|---|
| 1 | Drawing vacuum | To remove the air present in order to obtain an even distribution of vapour. |
| 2 | Steam injection | |
| 3 | Drawing vacuum | |
| 4 | Steam injection | |
| 5 | Drawing vacuum | |
| 4 | Steam injection | Final steam introduction phase |
| 5 | Temperature increase | Increasing the pressure until the sterilization temperature is reached (see Table 3) |
| 6 | Sterilize | Sterilization phase started as soon as the sterilization temperature was reached. The temperature was held at the sterilization temperature for the holding time (see Table 3). |
| 7 | Pressure relief | Pressure relief of the chamber in preparation for the drying phase, which takes place under vacuum. |
| 8 | Dry | Start of the drying phase by drawing vacuum. Vacuum applied for the drying time (see Table 3). |
| 9 | Ventilate | Filtered air was introduced into the chamber to remove the vacuum and prepare the chamber for relaxation. |
| 10 | Relax | Reaching the pressure area where the chamber door is automatically opened and the process is finished |

**Table 23: Conditions of the process of Example 6**

| | **Sample 15 (untreated)** | **Sample 16 (invention)** | **Sample 17 (invention)** |
|---|---|---|---|
| Sterilization temperature [°C] | n/a | 62 | 62 |
| Holding time [minute] | n/a | 1 | 2 |
| Drying time [minute] | n/a | 3 | 2 |

The contamination of the plant material was then assessed in accordance with the European Pharmacopoeia (paragraph 2.6.12 for TAMC and TYMC and paragraph 2.6.31 for Gram-negative bacteria that tolerate bile salts, E. coli and Salmonella). The results are provided in Table 24.

**Table 24: Contamination of the plant materials obtained after the process of Example 6**

| | **Sample 15 (untreated)** | **Sample 16 (invention)** | **Sample 17 (invention)** |
|---|---|---|---|
| TAMC test (cfu/g) | 45000 | 13000 | <1000 |
| TYMC test (cfu/g) | 25000 | 12000 | 1400 |
| Escherichia coli (per g) | Absent | Absent | Absent |
| Salmonella (per 25 g) | Absent | Absent | Absent |
| Gram-negative bacteria that tolerate bile salts (cfu/g) | <100 and >10 | <10 | <10 |

The microbial decontamination was effective for all samples of the invention.

The amounts of cannabinoid compounds were determined in accordance with the European Pharmacopoeia (paragraph 2.2.29). The results are provided in Table 25.

**Table 25: Cannabinoids content in the plant materials sterilized in Example 6**

| | **Sample 15 (untreated)** | **Sample 16 (invention)** | **Sample 17 (invention)** |
|---|---|---|---|
| Cannabinol (CBN) [g/100g] | <0,1 | <0,1 | <0,1 |
| (-)-trans-Δ⁹-tetrahydrocannabinol (Δ⁹-THC) [g/100g] | 0,4 | 0.5 | 0.5 |
| Tetrahydrocannabinolic acid (THCA) [g/100g] | 23.5 | 23.6 | 24.2 |
| Cannabidiol (CBD) [g/100g] | <0.1 | <0.1 | <0,1 |
| Cannabidiolic acid [g/100g] | <0.1 | <0.1 | <0.1 |
| Total Δ⁹-THC+ THCA [g/100g] | 23.9 | 23.6 | 24.7 |
| Total CBD + cannabidiolic acid [g/100g] | <0.1 | <0.1 | <0.1 |

No decarboxylation of THCA was observed in the samples of the invention.

The amounts of terpenes were determined in accordance with the European Pharmacopoeia (paragraph 2.2.28). The results are provided in Table 26.

**Table 26: Terpenes content in the plant materials sterilized in Example 6**

| **Terpenes** | **Sample 15 (untreated)** | **Sample 16 (invention)** | **Sample 17 (invention)** |
|---|---|---|---|
| α-Pinene [mg/g] | 0.15 | 0.10 | 0.10 |
| Camphene [mg/g] | 0.04 | 0.028 | 0.028 |
| β-Pinene [mg/g] | 0.27 | 0.18 | 0.18 |
| Myrcene[mg/g] | 0.81 | 0.34 | 0.35 |
| Δ-3- Carene [mg/g] | BDL¹⁾ | BDL¹⁾ | BDL¹⁾ |
| α-Terpinene [mg/g] | BDL¹⁾ | BDL¹⁾ | BDL¹⁾ |
| p-Cymene [mg/g] | BDL¹⁾ | BDL¹⁾ | BDL¹⁾ |
| Limonene [mg/g] | 0.78 | 0.44 | 0.44 |
| Eucalyptol (1,8-Cineol) [mg/g] | 0.068 | 0.067 | 0.070 |
| α-Ocimene [mg/g] | 0.011 | BDL¹⁾ | 0.013 |
| β-Ocimene [mg/g] | | | |
| γ-Terpinene [mg/g] | BDL¹⁾ | BDL¹⁾ | BDL¹⁾ |
| Terpinolene [mg/g] | BDL¹⁾ | 0.012 | 0.014 |
| Linalool [mg/g] | 1.2 | 1.1 | 1.0 |
| Isopulegol [mg/g] | BDL¹⁾ | BDL¹⁾ | BDL¹⁾ |
| Geraniol [mg/g] | BDL¹⁾ | BDL¹⁾ | BDL¹⁾ |
| β-Caryophyllene [mg/g] | 2.8 | 2.5 | 2.5 |
| α-Humulene [mg/g] | 0.98 | 0.90 | 0.92 |
| cis-Nerolidol [mg/g] | 1.3 | 1.2 | 1.3 |
| trans-Nerolidol [mg/g] | | | |
| Caryophyllenoxyde [mg/g] | 0.24 | 0.26 | 0.24 |
| Guaiol [mg/g] | 1.5 | 1.5 | 1.5 |
| α-Bisabolol [mg/g] | 1.1 | 1.1 | 1.1 |
| Total terpenes [mg/g] | 11 | 9.7 | 9.8 |

| | | | |
|---|---|---|---|
| 1) Below the Detection Limit | | | |

These results demonstrate that almost all terpenes present in the un-treated material were still detected in the cannabis flowers treated at 60°C (Samples 16 and 17). Even though the amounts of several terpenes were more or less reduced by the treatment, the overall terpenes content was retained in the samples treated according to the invention and all samples presented the characteristic odour of cannabis.

The drying loss was determined in accordance with the European Pharmacopoeia (paragraph 2.2.32). The drying loss of the untreated sample (Sample 15) and of the samples of the invention (Samples 16 and 17) was respectively of 5,8 %, 6,10 % and 6,7 %.

### Example 7: Effectiveness of invention process on different sample sizes

This example describes the procedure for validating different sample sizes (50 g, 500 g, 1kg and 2 kg). Samples of 1 kg are standard for microbiological germ reduction in the autoclave chamber. The aim is to prove that smaller and larger samples achieve the same microbiological results as the reference size of 1 kg.

The process was performed as described in any one of Examples 2 to 6, with the sample size, sterilization temperature, holding time and drying time provided in Table 27 below.

The contamination of the plant material was assessed by determining the TAMC after treatment in accordance with the European Pharmacopoeia (paragraph 2.6.12). In this case, only the TAMC test was determined, as it is the most reliable way to determine the change in the total bacterial count upon treatment.

**Table 27: description of the trials of Example 7 and microbiological counts.**

| **Program** | **Sample size [g]** | **Untreated material TAMC [cfu/g]** | **Result TAMC after treatment [cfu/g]** |
|---|---|---|---|
| Sterilization temperature 62°C, holding time 2 minutes, drying time: 3 minutes | 50 (Sample 18) | 860000 | 61000 |
| | 500 (Sample 19) | | 35000 |
| | 1000 (Sample 20) | | >30000 |
| | 2000 (Sample 21) | | 73000 |
| Sterilization temperature 72°C, holding time 2 minutes, drying time: 3 minutes | 50 (Sample 22) | 860,000 | 57000 |
| | 500 (Sample 23) | | 22000 |
| | 1000 (Sample 24) | | 100 |
| | 2000 (Sample 25) | | 3400 |
| Sterilization temperature 72°C, holding time 2 minutes, drying time: 3 minutes | 50 (Sample 26) | 860,000 | 4900 |
| | 500 (Sample 27) | | < 10 |
| | 1000 (Sample 28) | | 13000 |
| | 2000 (Sample 29) | | 5900 |
| Sterilization temperature 72°C, holding time 2 minutes, drying time: 3 minutes | 50 (Sample 30) | 860,000 | 360 |
| | 500 (Sample 31) | | 240 |
| | 1000 (Sample 32) | | < 10 |
| | 2000 (Sample 33) | | 14000 |
| Sterilization temperature 82°C, holding time 2 minutes, drying time: 3 minutes | 50 (Sample 34) | 860,000 | < 10 |
| | 500 (Sample 35) | | 40 |
| | 1000 (Sample 36) | | < 10 |
| | 2000 (Sample 37) | | 73 |

The results show - taking into account inhomogeneity and measurement uncertainty - a comparable germ reduction across all sample sizes. There is no significant difference between the quantities tested (50 g, 500 g, 1 kg and 2 kg in the paper bag). There is no discernible trend towards a systematically better or worse reduction for certain quantities.

## Claims

1. A process for producing a sterilized plant material comprising exposing a plant material to saturated steam at a sterilization temperature of less than 90°C for a holding time of 1 to 30 minutes in a chamber.

2. The process of claim 1, wherein the plant material is a plant material comprising terpenes, tetrahydrocannabinol and/or tetrahydrocannabinoic acid, preferably the plant material is of the Cannabaceae family, preferably the plant material is cannabis or hop, preferably the plant material is cannabis or hop flowers.

3. The process of claim 1 or 2, wherein the sterilization temperature is of less than 85°C, preferably of 82°C or less.

4. The process of any one of claims 1 to 3, wherein the sterilization temperature is of at least 60°C, preferably at least 70°C.

5. The process according to any one of claims 1 to 4, wherein the sterilization temperature is of 60 to 85°C, preferably of 62 to 82°C.

6. The process according to any one of claims 1 to 5, wherein the temperature is evenly distributed within the chamber.

7. The process according to any one of the preceding claims, wherein the cannabis is exposed to steam under reduced pressure, preferably under vacuum.

8. The process according to claim 7, wherein the following steps are performed:
a) introducing the plant material into the chamber;
b) drawing vacuum in the chamber containing the cannabis at least one time, preferably at least twice, preferably at least three times, optionally with the introduction of steam into the chamber between each time vacuum is drawn;
c) introducing steam into the chamber at a temperature below the sterilization temperature,
d) increasing the temperature in the chamber up to the sterilization temperature;
e) as soon as the sterilization temperature is reached, maintaining said temperature for the holding time; and
f) relieving the vacuum.

9. The process according to any one of the preceding claims, wherein the temperature is monitored during the holding time.

10. The process according to claim 9, wherein the process is stopped and the chamber is ventilated in case the temperature in the chamber exceeds a temperature defined as "sterilization temperature + 10°C".

11. The process according to any one of the preceding claims, comprising a step of vacuum drying after step e).

12. The process according to claim 11, wherein the vacuum drying is performed at reduced pressure, at a temperature below the sterilization temperature, preferably at a temperature 2 to 20°C below the sterilization temperature, preferably 5 to 10°C below the sterilization temperature.

13. The process according to claim 11, wherein the vacuum drying is performed by drawing the vacuum in the chamber.

14. The process according to any one of claims 11 to 13, wherein the drying is performed for a drying time of 1 to 30 minutes.

15. A sterilized plant material obtained or obtainable by the process according to any one of claims 1 to 14.
